# EUROPEAN PATENT SPECIFICATION

(11) **EP 3 332 243 B1**
(45) Date of publication and mention of the grant of the patent: **01.05.2024**
(21) Application number: 16833733.5
(22) Date of filing: 02.08.2016
(51) Int. Cl.: G01N 21/64, G01N 21/62, G01N 21/63, G01N 33/18

(54) **MULTI EXCITATION-MULTI EMISSION FLUOROMETER FOR MULTIPARAMETER WATER QUALITY MONITORING**
FLUORIMETER MIT MEHRFACHERREGUNG UND MEHRFACHEMISSION FÜR MULTIPARAMETRISCHE ÜBERWACHUNG DER WASSERQUALITÄT
FLUORIMÈTRE À EXCITATIONS MULTIPLES ET À ÉMISSIONS MULTIPLES PERMETTANT DE SURVEILLER LA QUALITÉ DE L'EAU À PARAMÈTRES MULTIPLES

(30) Priority: 03.08.2015 US 201562200336 P
(43) Date of publication of application: 13.06.2018
(73) Proprietor: YSI, Inc., Yellow Springs, OH 45387 (US)
(72) Inventor: FLANAGAN, Kevin R., Yellow Springs, Ohio 45387 (US); PALASSIS, Christopher J, Yellow Springs, Ohio 45387 (US)
(74) Representative: DREISS Patentanwälte PartG mbB
(86) International application number: PCT/US2016/045152
(87) International publication number: WO 2017/023925

(56) References cited:
- WO-A1-03/023379
- WO-A2-2005/008198
- CN-A- 103 245 644
- CN-A- 104 730 054
- US-A- 4 293 225
- US-A- 6 124 597
- US-A1- 2005 070 005
- US-A1- 2013 327 961
- US-B1- 6 233 047
- US-B1- 7 470 917
- US-B2- 7 129 505
- US-B2- 7 578 973
- ROBERTO BARBINI ET AL: "ENEA fluorosensor system used in monitoring the Adriatic Sea", PROCEEDINGS OF SPIE, vol. 2959, 17 January 1997 (1997-01-17), pages 254-264, XP055528749, 1000 20th St. Bellingham WA 98225-6705 USA ISSN: 0277-786X, DOI: 10.1117/12.264276

## Description

### BACKGROUND OF THE INVENTION

### 1 . Field of Invention

This invention relates to a technique for determining the quality of water; and more particularly relates to a technique for determining the quality of water based upon the detection of multiple coexisting fluorescent species present in the water.

### 2. Description of Related Art

Techniques for monitoring water are known in the art, including monitoring for the presence of sewage and waste water. A confirmation of sewage impacted water is a complicated process, e.g., especially when using a single emission wavelength alone, which has been found to not unambiguously determine the presence waste water. In view of this, there is a need in the industry for a better way for monitoring water.

From US 2013/0327961 A1 a fluorometer is known comprising several emission detectors and a correspondant number of photodiodes.

CN 103245644 A describes a fluorometer comprising light sources and an echell gratimng aoptical splitter and a gate time delay ICCD detector. Further, it comprises a sample cell and a pump for providing sample water into the sample cell.

WO 2005/008198 A2 describes a compact interference imaging spectrometer.

### SUMMARY OF THE INVENTION

The present invention concerns techniques for monitoring the quality of water.

The invention is defined by independent claim 1 and includes an apparatus, in the form of a fluorometer, for monitoring the quality of water, featuring a combination of an array of excitation sources, an array of multiple emission detectors and a signal processor or processing module.

Each excitation source in the array of excitation sources is configured to provide respective excitation source optical signaling at a respective illuminating wavelength, e.g., in relation to the water being monitored.

The array of multiple emission detectors is configured to detect multiple emission wavelengths emitted from the water containing information about multiple coexisting fluorescent species present in the water that emit optical radiation at at least two different wavelengths when illuminated by the respective illuminating wavelength provided from the array of excitation sources, and provide multiple emission detector signaling containing information about the multiple coexisting fluorescent species.

The signal processor or processing module is configured to receive the multiple emission detector signaling, and determine corresponding signaling containing information about an identification of the multiple coexisting fluorescent species present in the water using a near-simultaneous identification technique, based upon the multiple emission detector signaling received.

The apparatus may include one or more of the following additional features:
The array of excitation sources may include an excitation source, e.g., like an excitation LED, and the illuminating wavelength may be 280 nanometers. Outside the scope of the present invention, the array of multiple emission detectors may include a first emission detector configured to detect the optical radiation at 340 nanometers for detecting the present of peak-T, protein-like (e.g., including peak T-tryptophan) in the water; and a second emission detector configured to detect the optical radiation at 450 nanometers for detecting the present of peak A humic/fulvic-like in the water.

In non-claimed embodiments, the array of multiple emission detectors may include a plurality of photodiodes and optical bandpass filters configured to sense and filter the multiple emission wavelengths emitted from water, and provide the multiple emission detector signaling.

The optical bandpass filters may include, e.g., a first photodiode and optical bandpass filter configured to filter the optical radiation at 340 nanometers for detecting the present of peak-T, protein-like in the water; and a second photodiode and optical bandpass filter configured to filter the optical radiation at 450 nanometers for detecting the present of peak A humic/fulvic-like in the water.

The array of excitation sources may include a plurality of excitation sources configured to provide a plurality of excitation source optical signaling at a plurality of illuminating wavelengths, e.g., such as plurality of excitation LEDs.

In non-claimed embodiments, the array of multiple emission detectors may include optical bandpass filters spectrally centered about fluorescence emission wavelengths of interest.

The array of multiple emission detectors of the invention includes a combination of a focusing lens and an optical spectrum analyzer for fluorescence capture and analysis.

The plurality of excitation sources may be configured to respond to suitable control signaling and near-simultaneously provide the plurality of excitation source optical signaling to produce the plurality of illuminating wavelengths and detect the multiple emission wavelengths. Alternatively, the plurality of excitation sources may be configured to respond to corresponding suitable control signaling and selectively provide the plurality of excitation source optical signaling to produce the plurality of illuminating wavelengths and detect the multiple emission wavelengths. In other words, the plurality of excitation sources and the array of multiple emission detectors may be configured to respond to control signaling and either near-simultaneously or selectively provide the plurality of excitation source optical signaling to produce any combination of excitation wavelengths or detected fluorescence emission.

The fluorometer is configured in, or forms part of, a single sensor body. The single sensor body includes, or take the form of, a sonde having a water tight housing that encloses the fluorometer. The sonde may include a port; and the fluorometer may include an electrical connector configured to plug into the port of the sonde. The electrical connector may be configured to attach to a printed circuit board (PCB), e.g., containing sensor electronics. The sensor electronics may include the signal processor or processing module. The fluorometer may include an opto-mechanical head that contains electro-opto-mechanical components, including the array of excitation sources and the array of multiple emission detectors. The water tight housing may include a window configured to allow optical transmission/interaction between the multiple coexisting fluorescent species to be measured in the water being monitored and the electro-opto-mechanical components contained in the sonde. By way of example, the window may be made of Sapphire, as well as multiple other window materials.

The signal processor or processing module is configured to provide the corresponding signaling containing information about the identification of the multiple coexisting fluorescent species present in the water using the near-simultaneous identification technique for further processing. By way of example, the further processing may include, or take the form of, providing control signaling for further processing the water being monitored; or the further processing may include providing the control signaling for adapting the water monitoring process itself for monitoring the water. By way of further example, the corresponding signaling may include information to provide a visual display related to the identification, and/or an audio/visual alarm, etc.

The fluorometer may include an opto-mechanical head configured with electro-opto-mechanical components, including the array of excitation sources and the array of multiple emission detectors.

The plurality of excitation sources is configured or arranged circumferentially about the array of multiple emission detectors.

At the time of the instant patent application filing, others similar products are known and made by companies like Turner Designs and UviLux Tryptophan Fluorometer.

Similarities between the present invention and these known products may include: Fluorescence-based optical sensing of wastewater, emission wavelength for Tryptophan will overlap with only one of the emission wavelengths set forth herein.

Differences between the present invention and these known products may include: The sensor set forth herein according to the present invention has a key advantage and innovation of utilizing dual emission wavelengths for meaningful and increased confidence of detection of wastewater - all in a single sensing body.

### BRIEF DESCRIPTION OF THE DRAWING

The drawing includes Figures 1 - 4, which are not necessarily drawn to scale, as follows:
Figure 1 shows a diagram of apparatus in the form of a sensor body.
Figure 2 includes Figures 2A and 2B, where Figure 2A is a front view of an opto-mechanical head that may form part of the sensor body in Figure 1, and where Figure 2B is a cross-sectional (or cutaway) view of the opto-mechanical head in Figure 2A, showing a fluorometer outside the scope of the present invention.
Figure 3 includes Figures 3A and 3B, where Figure 3A is a front view of an opto-mechanical head for multiple parameter sensing that may form part of the sensor body in Figure 1, and where Figure 3B is a cross-sectional view of the opto-mechanical head in Figure 3A, according to some embodiments of the present invention.
Figure 4 shows a block diagram of apparatus, e.g., having a signal processor or signal processing module for implementing signal processing functionality.

### DETAILED DESCRIPTION OF BEST MODE OF THE INVENTION

The Underlying Technique in General In a first example, fluorometer generally indicated as 20 may be configured to measure fluorescence of peak T-tryptophan-like (λ_{ex/em} = 280/340nm) and peak A humic/fulvic-like (λ_{ex/em} = 280/450nm), e.g., using a single excitation source / dual emission detection as means of identifying sewage impacted water in general. The affirmative confirmation of sewage impacted water is complicated in that it may be more accurately determined through near-simultaneous identification of multiple fluorescence species. For the particular case at hand, and according to the present invention, one may seek to near-simultaneously identify two species requiring two detected fluorescence emission wavelengths within a single sensing body. It is the combined information of multiple fluorescence that serves to address the single issue of wastewater identification. The inventors have come to understand that a single emission wavelength alone cannot unambiguously determine the presence wastewater, and provide new and unique techniques disclosed herein to solve this "single emission wavelength" problem in the art.

Moreover, the present invention is not intended to be restricted to the identification of only two fluorescence species, but rather is intended to encompass the possibility of near-simultaneous detection of multiple fluorescence species, e.g., including three or more fluorescence species. According to the invention, this notion is extended to include multiple excitation sources and multiple emission wavelength detection to near-simultaneously detect multiple fluorescence species within a single sensing body. For water quality monitoring, it is often the case that the presence of multiple fluorescence species tends to obscure or interfere with any particular desired measurand. The near-simultaneous identification of the multiple species disclosed or presented herein serves to isolate and more singly describe/identify the water quality parameter of interest.

### Figures 1-3

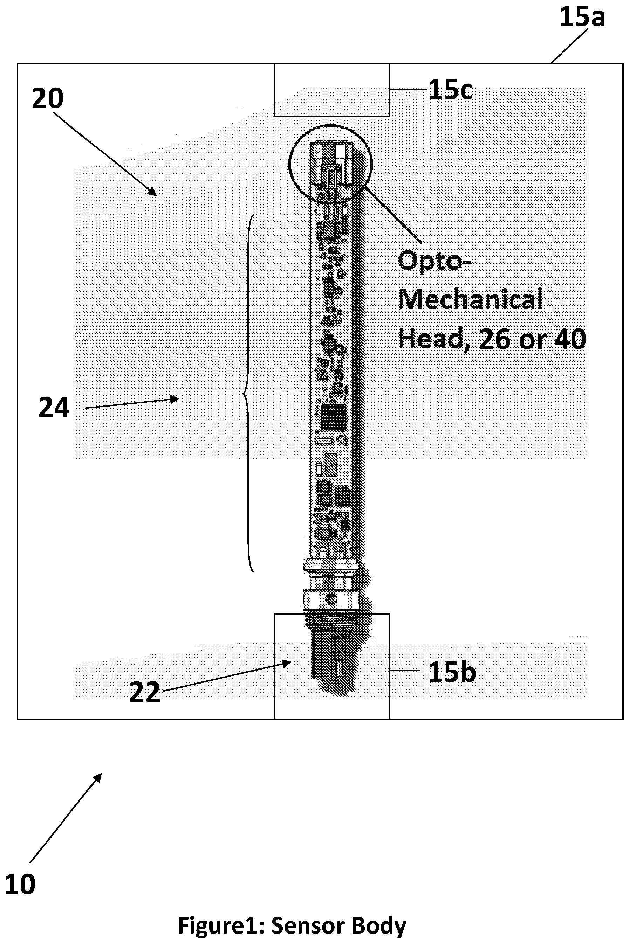

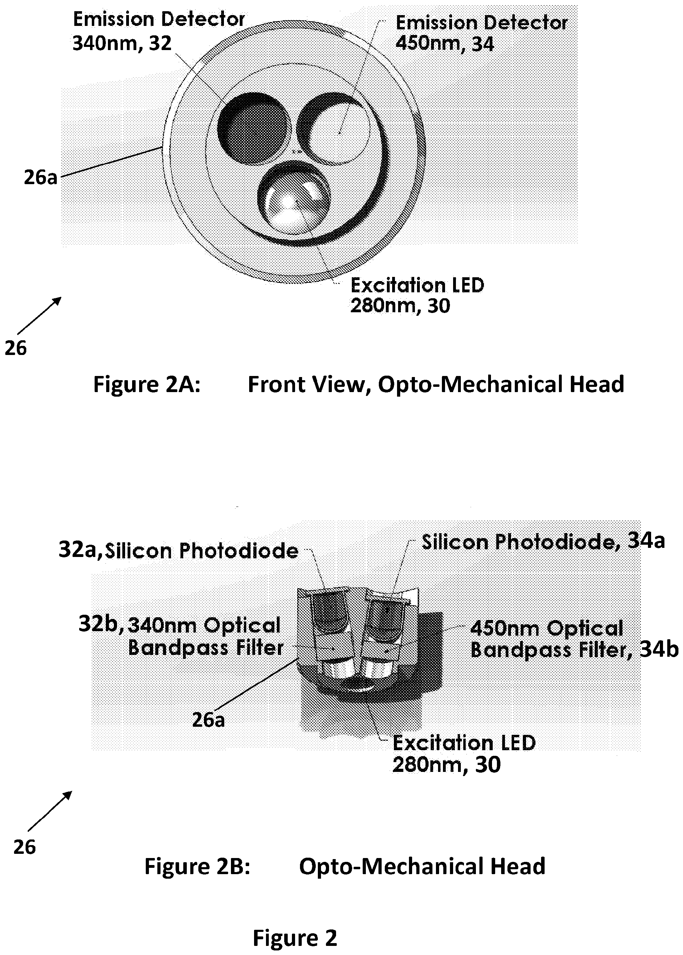

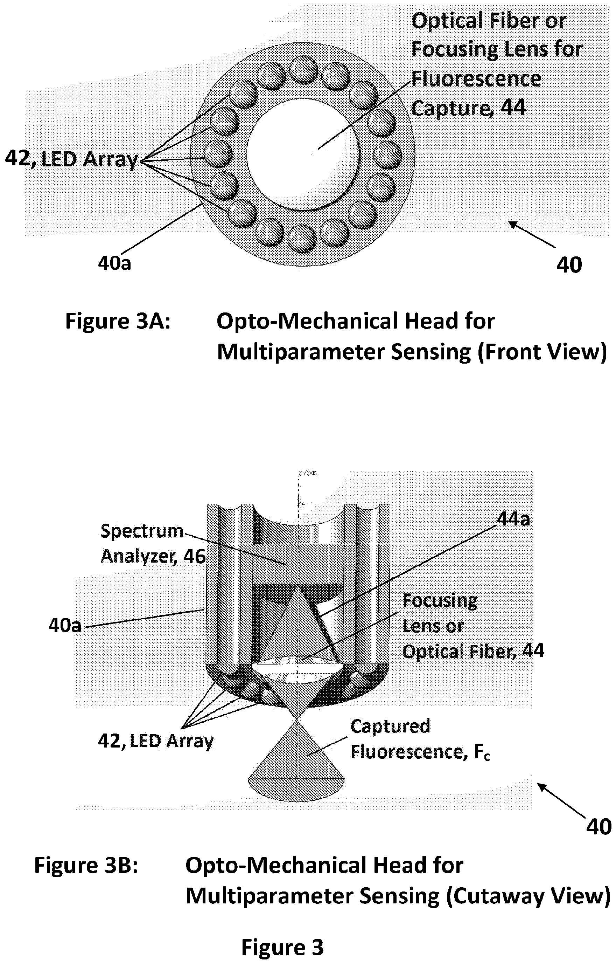

Figures 1 and 2 shows a first example outside the scope of the present invention, based upon one seeking to near-simultaneously identify two species requiring two detected fluorescence emission wavelengths within a single sensing body, e.g., which may take the form of apparatus 10 generally shown in Figure 1 having a fluorometer 20 with an opto-mechanical head 26 shown in detail in Figure 2. This notion can be extended to include multiple excitation sources and multiple emission wavelength detection to near-simultaneously detect multiple fluorescence species within a single sensing body using an opto-mechanical head 40, e.g. consistent with that disclosed in relation to Figure 3.

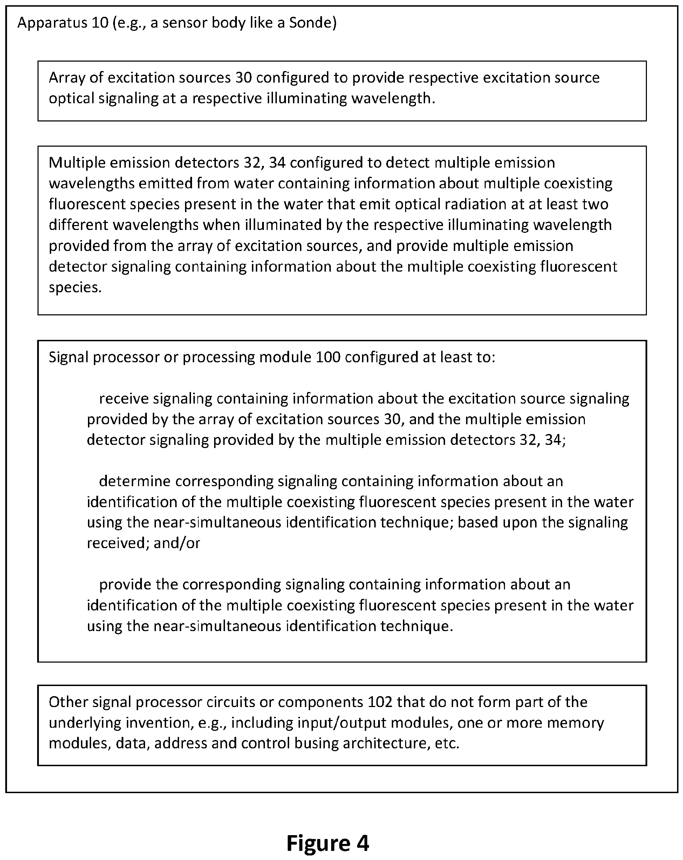

The implementations of the sensors or sensing bodies 10 and the fluorometers 20 differ primarily in the details concerning the opto-mechanical heads 26 and 40 shown in Figures 2 and 3. The sensors or sensor bodies 10 disclosed in this patent application have at least the following in common: The sensor body 10 generally includes, or consists of, a water tight housing 15a (Figure 1) that encloses the fluorometer 20 and has at least part of an electrical connector 22 that plugs into a port 15b on the main sensor body 10. The sensors or sensing bodies 10 include, or take the form of, a Sonde structure. The fluorometer 20 may be configured with a printed circuit board (PCB) generally indicated as 24, and the electrical connector 22 may also be attached to the printed circuit board (PCB) 24 containing the sensor electronics, e.g., which may include a signal processor or processing module like element 100 (Figure 4), e.g., for implementing signal processing functionality consistent with that disclosed herein. The fluorometer 20 may be configured with the opto-mechanical head like elements 26 or 40, which may be attached to the PCB 24. The opto-mechanical head like elements 26 or 40 may contain the electro-opto-mechanical components, e.g., including light emitting diodes (LEDs) like element 30 and emission detectors like elements 32, 34 having photodetectors (PDs) like elements 32a, 34a and optical bandpass filters 32b, 34b. One end/side of the water tight housing 15a may also contain a window 15c (Figure 1) that may be configured to allow optical transmission/interaction between the fluorophore (i.e., fluorescent species to be measured) and the optical sensing components like elements 30, 32 and 34 in relation to the embodiment in Figure 2, or elements 42 or 44 in relation to the embodiment in Figure 3. By way of example, the window may be made of Sapphire, although the scope of the invention is not intended to be limited to the same. Embodiments are envisioned using other types or kind of window material either now known or later developed in the art, e.g., as one skilled in the art would be appreciate.

In particular, Figure 1 shows or depicts the single sensor body 10 with the electrical connection 22 at its bottom, the PCB 24 (e.g., shown in Figure 1 as an electrically populated circuit board in the main body of the sensor 10), and the opto-mechanical head like element 26 or 40 (as circled in Figure 1), e.g., containing the LEDs like elements 30 (Figure 2), PDs and optical bandpass filters like elements 32, 34 as disclosed in relation to Figure 2.

In Figure 1, the sensor body 10 is shown by way of example as a representation of a typical sensor body and is not intended to be accurate in scale or engineering detail per se. One of the essential components which differentiates all of the disclosed embodiments herein is the opto-mechanical head 26 or 40 (as circled in Figure 1). In view of this, and to that end, Figures 2A, 2B, 3A and 3B show only details associated with the opto-mechanical head 26 or 40.

### Figure 2: Example of Particular Embodiment

Figures 2A and 2B show a first embodiment of the opto-mechanical head 26 that can form part of a sensor like element 10 (Figure 1), outside the scope of the present invention. By way of example, the opto-mechanical head 26 includes an opto-mechanical head body 26a that may contain a single LED like element 30 at an excitation wavelength of 280nm, and two emission detectors like elements 32, 34. By way of example, the two emission detectors 32, 34 may include two Silicon or other suitable Photodetectors 32a, 34a with respective optical bandpass filters 32b, 34b spectrally centered at 340nm and 450nm. This opto-mechanical configuration is designed to detect two coexisting fluorescent species that emit optical radiation at 340nm and 450nm respectively when illuminated by the 280nm optical source like element 30. By way of example, the photodiodes 32a, 34a and the LED 30 may be configured, or may employ, a ball lens configuration to maximize fluorescence collection, e.g., consistent with that shown in Figures 2A and 2B.

### Figure 3: Example of Generalized Embodiment

Figures 3A and 3B show a second, more generalized, embodiment having the opto-mechanical head 40 having an opto-mechanical head body 40a that can form part of the sensor like element 10 (Figure 1), according to some embodiments of the present invention. By way of example, the opto-mechanical head 40 may contain an array 42 of many excitation LEDs. In Figure 3A, the array 42 is shown having 16 excitation LEDs, although the scope of the invention is not intended to be limited to any particular number of excitation LEDs. The excitation wavelengths and number of LEDs can be chosen to suit the desired application. For example, depending on the particular application a different number of excitation LEDs may be used. In operation, each excitation LED is configured to provide respective excitation LED optical signaling at a respective illuminating wavelength, e.g., consistent with that set forth herein. Moreover, the opto-mechanical head 40 may include receiving optics 44, e.g., such as, outside the scope of the invention, an array of photodiodes with associated optical bandpass filters spectrally centered about fluorescence emission wavelengths of interest, or, in the invention, an optical spectrum analyzer like element 46 as shown (Figure 3B). Both of these receiving optics techniques serve as a means to spectrally discriminate the collected/captured fluorescence optical signaling generally indicated as F_{c}. The fluorescence, in the invention, is captured through a focusing lens like element 44 (Figure 3B) that provides focusing lens optical signaling 44a onto a spectrum analyzer like element 46, or, in non-claimed embodiments, by using one or more fiber optic waveguides, e.g., including a bundle of optical fibers (also indicated by reference label 44). The opto-mechanical configuration 40 may be configured or designed to detect multiple, independent or coexisting fluorescent species that emit optical radiation in a range or distribution of emission wavelengths when illuminated by the LED array 42. The array of LEDs 42 and photodiodes (or, as in the invention, the spectrum analyzer 46) need not be near-simultaneously activated, but can be selectively enabled or scanned to produce any combination of excitation wavelengths or detected fluorescence emission.

In Figure 3, the plurality of LED excitation sources 42 is configured or arranged circumferentially about the array of multiple emission detectors 44.

Figure 4: Implementation of Signal Processing Functionality By way of further example, Figure 4 shows the apparatus or sensor body 10 for implementing the associated signal processing functionality. The apparatus or sensor body 10 may include a signal processor or processing module 100 configured at least to:
receive signaling containing information about excitation source signaling provided by an array of excitation sources, each excitation source configured to provide respective excitation source optical signaling at a respective illuminating wavelength, and multiple emission detector signaling provided by an array of multiple emission detectors configured to detect multiple emission wavelengths emitted from water containing information about multiple coexisting fluorescent species present in the water that emit optical radiation at at least two different wavelengths when illuminated by the respective illuminating wavelength provided from the array of excitation sources, the multiple emission detector signaling containing information about the multiple coexisting fluorescent species; and
determine corresponding signaling containing information about an identification of the multiple coexisting fluorescent species present in the water using a near-simultaneous identification technique, based upon the signaling received.

In operation, the signal processor or processing module 100 may be configured to provide the corresponding signaling containing information about the identification of the multiple coexisting fluorescent species present in the water using the near-simultaneous identification technique, e.g., for further processing, consistent with that set forth herein. The scope of the invention is not intended to be limited to any particular type, kind or manner of further processing, and may include further processing techniques either now known or later developed in the future.

The signal processor or processing module 100 may be configured in, or form part of, a sensor body, e.g., like a sonde.

By way of example, the functionality of the signal processor or processing module 100 may be implemented using hardware, software, firmware, or a combination thereof. In a typical software implementation, the signal processor or processing module 100 would include one or more microprocessor-based architectures having, e. g., at least one signal processor or microprocessor like element 100. One skilled in the art would be able to program with suitable program code such a microcontroller-based, or microprocessor-based, implementation to perform the signal processing functionality disclosed herein without undue experimentation. For example, the signal processor or processing module 100 may be configured, e.g., by one skilled in the art without undue experimentation, to receive the signaling containing information about excitation source signaling provided by an array of excitation sources, each excitation source configured to provide respective excitation source optical signaling at a respective illuminating wavelength, and multiple emission detector signaling provided by multiple emission detectors configured to detect multiple emission wavelengths emitted from water containing information about multiple coexisting fluorescent species present in the water that emit optical radiation at at least two different wavelengths when illuminated by the respective illuminating wavelength provided from the array of excitation sources, the multiple emission detector signaling containing information about the multiple coexisting fluorescent species, consistent with that disclosed herein.

Moreover, the signal processor or processing module 100 may be configured, e.g., by one skilled in the art without undue experimentation, to determine the corresponding signaling containing information about an identification of the multiple coexisting fluorescent species present in the water using a near-simultaneous identification technique, consistent with that disclosed herein. By way of example, the scope of the invention is not intended to be limited to any particular type or kind of signal processing implementation and/or technique for the near-simultaneous identification of the multiple coexisting fluorescent species present in the water. The scope of the invention is intended to include signal processing implementations and/or techniques for the near-simultaneous identification of the multiple coexisting fluorescent species present in the water that are known by one skilled in the art.

The signal processor or processing module 10 may also include, e.g., other signal processor circuits or components 102, including random access memory or memory module (RAM) and/or read only memory (ROM), input/output devices and control, and data and address buses connecting the same, and/or at least one input processor and at least one output processor, e.g., which would be appreciated by one skilled in the art.

### The Scope of the Invention

While the invention has been described with reference to an exemplary embodiment, it will be understood by those skilled in the art that various changes may be made and equivalents may be substituted for elements thereof without departing from the scope of the invention as defined by the appended claims.

## Claims

1. A fluorometer (20) for monitoring the quality of
water, **characterized by** comprising:
an array (42) of excitation sources, each excitation source configured to provide respective excitation source optical signaling at a respective illuminating wavelength; an array of multiple emission detectors configured to detect multiple emission wavelengths emitted from water containing information about multiple coexisting fluorescent species present in the water that emit optical radiation at at least two different wavelengths when illuminated by the respective illuminating wavelength provided from the array (42) of excitation sources, and provide multiple emission detector signaling containing information about the multiple coexisting fluorescent species; and
a signal processor or processing module (100) configured to receive the multiple emission detector signaling, and determine corresponding signaling containing information about an identification of the multiple coexisting fluorescent species present in the water using a near-simultaneous identification technique, based upon the multiple emission detector signaling received,
wherein the fluorometer (20) is configured in, or forms part of, a single sensor body, and
wherein the single sensor body comprises a sonde having a water tight housing (15a) that encloses the fluorometer (20);
**characterized in that**:
the array of multiple emission detectors includes a combination of a focusing lens (44) and an optical spectrum analyzer (46),
wherein the focusing lens (44) focuses lens optical signaling (44a) to the spectrum analyzer (46), and,
wherein the array (42) of excitation sources is arranged circumferentially about the optical spectrum analyzer (46)

2. A fluorometer (20) according to claim 1, wherein the array of excitation sources (42) comprises an excitation source, and the illuminating wavelength is 280 nanometers.

3. A fluorometer (20) according to claim 2, wherein the excitation source comprises an excitation LED.

4. A fluorometer (20) according to claim 1, wherein the plurality of excitation sources and the optical spectrum analyzer (46) are configured to respond to control signaling and either near-simultaneously or selectively provide the plurality of excitation source optical signaling to produce any combination of excitation wavelengths or detected fluorescence emission.

5. A fluorometer (20) according to claim 1, wherein the sonde comprises a port; and the fluorometer comprises an electrical connector configured to plug into the port of the sonde.

6. A fluorometer (20) according to claim 5, wherein the electrical connector is configured to attach to a printed circuit board (24) containing sensor electronics.

7. A fluorometer (20) according to claim 6, wherein the sensor electronics include the signal processor or processing module (100).

8. A fluorometer (20) according to claim 6, wherein the fluorometer (20) comprises an opto-mechanical head (26, 40) that contains electro-opto-mechanical components, including the array of excitation sources (42) and the array (44) of multiple emission detectors (44).

9. A fluorometer (20) according to claim 8, wherein the water tight housing (15a) comprises a window (15c) configured to allow optical transmission/interaction between the multiple coexisting fluorescent species to be measured and the electro-opto-mechanical components, including where the window is made of Sapphire.

## Patentansprüche

1. Fluorometer (20) zum Überwachen der Wasserqualität, **gekennzeichnet dadurch, dass** es umfasst:
ein Array (42) von Anregungsquellen, wobei jede Anregungsquelle konfiguriert ist, um eine jeweilige optische Signalisierung der Anregungsquelle bei einer jeweiligen Beleuchtungswellenlänge bereitzustellen; ein Array von mehreren Emissionsdetektoren, das konfiguriert ist, um mehrere Emissionswellenlängen zu detektieren, die von Wasser emittiert werden, die Informationen über mehrere koexistierende fluoreszierende Spezies enthalten, die in dem Wasser vorhanden sind, die optische Strahlung bei mindestens zwei unterschiedlichen Wellenlängen emittieren, wenn sie durch die jeweilige Beleuchtungswellenlänge beleuchtet werden, die von dem Array (42) von Anregungsquellen bereitgestellt wird, und mehrere Emissionsdetektorsignalisierungen bereitstellen, die Informationen über die mehreren koexistierenden fluoreszierenden Spezies enthält; und
einen Signalprozessor oder ein Verarbeitungsmodul (100), der/das konfiguriert ist, um die mehrere Emissionsdetektorsignalisierungen zu empfangen, und eine entsprechende Signalisierung zu bestimmen, die Informationen über eine Identifizierung der mehreren koexistierenden fluoreszierenden Spezies enthält, die in dem Wasser vorhanden sind, unter Verwendung einer nahezu gleichzeitigen Identifikationstechnik, basierend auf den empfangenen mehreren Emissionsdetektorsignalisierungen,
wobei das Fluorometer (20) in einen einzelnen Sensorkörper konfiguriert ist oder einen Teil davon ausbildet, und
wobei der einzelne Sensorkörper eine Sonde umfasst, die ein wasserdichtes Gehäuse (15a) aufweist, das das Fluorometer (20) umschließt;
**dadurch gekennzeichnet, dass**:
das Array von mehreren Emissionsdetektoren eine Kombination einer Fokussierlinse (44) und eines optischen Spektrumsanalysators (46) einschließt,
wobei die Fokussierlinse (44) die Signalisierung (44a) der optischen Linse zu dem Spektrumsanalysator (46) fokussiert, und
wobei das Array (42) von Anregungsquellen in Umfangsrichtung um den optischen Spektrumsanalysator (46) herum angeordnet ist.

2. Fluorometer (20) nach Anspruch 1, wobei das Array von Anregungsquellen (42) eine Anregungsquelle umfasst und die Beleuchtungswellenlänge 280 Nanometer beträgt.

3. Fluorometer (20) nach Anspruch 2, wobei die Anregungsquelle eine Anregungs-LED umfasst.

4. Fluorometer (20) nach Anspruch 1, wobei die Vielzahl von Anregungsquellen und der optische Spektrumsanalysator (46) konfiguriert sind, um auf die Steuersignalisierung zu reagieren und entweder nahezu gleichzeitig oder selektiv die Vielzahl von optischen Signalisierungen der Anregungsquellen bereitzustellen, um eine beliebige Kombination von Anregungswellenlängen oder detektierten Fluoreszenzemission zu erzeugen.

5. Fluorometer (20) nach Anspruch 1, wobei die Sonde einen Anschluss umfasst; und das Fluorometer einen elektrischen Verbinder umfasst, der konfiguriert ist, um in den Anschluss der Sonde hineingesteckt zu werden.

6. Fluorometer (20) nach Anspruch 5, wobei der elektrische Verbinder konfiguriert ist, um an einer Leiterplatte (24), die Sensorelektronik enthält, anzuschließen.

7. Fluorometer (20) nach Anspruch 6, wobei die Sensorelektronik den Signalprozessor oder das Verarbeitungsmodul (100) einschließt.

8. Fluorometer (20) nach Anspruch 6, wobei das Fluorometer (20) einen optomechanischen Kopf (26, 40) umfasst, der elektrooptomechanische Komponenten enthält, einschließlich des Arrays von Anregungsquellen (42) und des Arrays (44) von mehreren Emissionsdetektoren (44).

9. Fluorometer (20) nach Anspruch 8, wobei das wasserdichte Gehäuse (15a) ein Fenster (15c) umfasst, das konfiguriert ist, um eine optische Übertragung/Interaktion zwischen den mehreren zu messenden koexistierenden fluoreszierenden Spezies und den elektrooptomechanischen Komponenten zu ermöglichen, einschließlich wobei das Fenster aus Saphir hergestellt ist.

## Revendications

1. Fluoromètre (20) permettant de surveiller la qualité de l'eau, **caractérisé en ce qu'**il comprend:
un réseau (42) de sources d'excitation, chaque source d'excitation étant configurée pour fournir une signalisation optique de source d'excitation respective à une longueur d'onde d'éclairage respective ; un réseau de détecteurs d'émissions multiples configurés pour détecter des longueurs d'onde d'émissions multiples émises par l'eau contenant des informations sur de multiples espèces fluorescentes coexistantes présentes dans l'eau qui émettent un rayonnement optique à au moins deux longueurs d'onde différentes lorsqu'elles sont illuminées par la longueur d'onde d'éclairage respective fournie par le réseau (42) de sources d'excitation, et fournir une signalisation de détecteurs d'émissions multiples contenant des informations sur les multiples espèces fluorescentes coexistantes; et
un module de traitement ou processeur de signal (100) configuré pour recevoir la signalisation de détecteur d'émissions multiples, et déterminer une signalisation correspondante contenant des informations sur une identification des multiples espèces fluorescentes coexistantes présentes dans l'eau à l'aide d'une technique d'identification quasi simultanée, sur la base de la signalisation de détecteur d'émissions multiples reçue,
dans lequel le fluoromètre (20) est configuré dans un corps de capteur unique, ou en fait partie, et
dans lequel le corps de capteur unique comprend une sonde ayant un boîtier (15a) étanche à l'eau qui renferme le fluoromètre (20);
**caractérisé en ce que**:
le réseau de détecteurs d'émissions multiples comporte une combinaison d'une lentille de focalisation (44) et d'un analyseur de spectre optique (46),
dans lequel la lentille de focalisation (44) focalise la signalisation optique de lentille (44a) vers l'analyseur de spectre (46), et,
dans lequel le réseau (42) de sources d'excitation est agencé circonférentiellement autour de l'analyseur de spectre optique (46).

2. Fluoromètre (20) selon la revendication 1, dans lequel le réseau de sources d'excitation (42) comprend une source d'excitation, et la longueur d'onde d'éclairage est de 280 nanomètres.

3. Fluoromètre (20) selon la revendication 2, dans lequel la source d'excitation comprend une DEL d'excitation.

4. Fluoromètre (20) selon la revendication 1, dans lequel la pluralité de sources d'excitation et l'analyseur de spectre optique (46) sont configurés pour répondre à une signalisation de commande et pour fournir, soit de manière quasi simultanée, soit de manière sélective, une signalisation optique de pluralité de sources d'excitation pour produire toute combinaison de longueurs d'onde d'excitation ou d'émission de fluorescence détectée.

5. Fluoromètre (20) selon la revendication 1, dans lequel la sonde comprend un port; et le fluoromètre comprend un connecteur électrique configuré pour se brancher dans le port de la sonde.

6. Fluoromètre (20) selon la revendication 5, dans lequel le connecteur électrique est configuré pour se fixer à une carte de circuit imprimé (24) contenant une électronique de capteur.

7. Fluoromètre (20) selon la revendication 6, dans lequel l'électronique de capteur comporte le module de traitement ou processeur de signal (100).

8. Fluoromètre (20) selon la revendication 6, dans lequel le fluoromètre (20) comprend une tête opto-mécanique (26, 40) qui contient des composants électro-opto-mécaniques, y compris le réseau de sources d'excitation (42) et le réseau (44) de détecteurs d'émissions multiples (44).

9. Fluoromètre (20) selon la revendication 8, dans lequel le boîtier (15a) étanche à l'eau comprend une fenêtre (15c) conçue pour permettre une interaction/transmission optique entre les multiples espèces fluorescentes coexistantes à mesurer et les composants électro-opto-mécaniques, y compris lorsque la fenêtre est faite en saphir.
